# EUROPEAN PATENT SPECIFICATION

(11) **EP 4 472 522 B1**
(45) Date of publication and mention of the grant of the patent: **29.10.2025**
(21) Application number: 23714602.2
(22) Date of filing: 14.03.2023
(51) Int. Cl.: A61B 10/02

(54) **GUN FOR BIOPSIES**
BIOPSIEPISTOLE
PISTOLET POUR BIOPSIES

(30) Priority: 18.03.2022 IT 202200005303
(43) Date of publication of application: 11.12.2024
(73) Proprietor: Musicco, Francesca, 25123 Brescia (IT); Musicco, Nicola, 25123 Brescia (IT)
(72) Inventor: RAMORINO, Giorgio, 25123 Brescia (IT); MUSICCO, Francesca, 25123 Brescia (IT); MUSICCO, Nicola, 25123 Brescia (IT)
(74) Representative: Pes, Matteo
(86) International application number: PCT/IB2023/052434
(87) International publication number: WO 2023/175486

(56) References cited:
- DE-A1- 102008 038 414
- US-A1- 2009 299 221
- US-B1- 6 283 925

## Description

### Field of the invention

Object of the present invention is a gun for biopsies, in particular an automatic or semi-automatic gun of the kind used to sample tissues from the human body.

### Known art

Guns for biopsy are used to sample soft tissue inside the human body, usually to carry out histological tests.

The present invention refers to an automatic gun that leverages a guillotine-like technique to take a soft tissue sample from a patient's body, e.g. kidney, prostate, breast tissues.

Generally, disposable automatic guns for biopsy comprise a box-shaped body with which a needle, usually disposable, can be removably combined. The box-shaped body has a substantially parallelepiped shape so that it can be held with only one hand by the healthcare professional carrying out the biopsy. For this reason in the box-shaped body a first distal end can be identified, corresponding to the end closest to the patient during the biopsy and farthest from the healthcare professional holding the gun, and a proximal end corresponding to the end furthest from the patient during the biopsy and closest to the healthcare professional holding the gun. The needle extends in a cantilevered way from the distal end of the box-shaped body, e.g. for 10-15 cm, so that it can be inserted into the patient's body.

The needle comprises two components:
- a cannula provided with an echogenic tip and an end constrained to a first carriage which is slidably housed in the box-shaped body;
- a stylet coaxially housed in the cannula and sliding therein. The stylet has a pointed end at which a hollow for collecting the tissue sample is provided. The opposite end of the stylet is constrained to a second carriage which is slidably housed in the box-shaped body.

The first and second carriages translate in the box-shaped body between an initial retracted position, at the proximal end of the box-shaped body, and a forward end position near the distal end of the box-shaped body. Corresponding springs apply the necessary thrust to the carriages to translate between these two positions.

The needle defines the longitudinal axis of the box-shaped body; the first and second carriages move along the longitudinal axis, if the gun is configured with the coaxial carriages, or move parallel to the longitudinal axis, if the gun is configured with side-by-side carriages moving on parallel axes.

The carriages remain normally locked in the respective retracted positions. A drive mechanism, defined *firing mechanism,* allows the healthcare professional to unlock the carriages as explained below.

The firing mechanism comprises a loading button for each of the two carriages and, therefore, comprises a first and a second loading button usually positioned on the top surface or side surface of the box-shaped body of the gun. The loading buttons are sliding with respect to the box-shaped body, parallel to the longitudinal axis, and act on the retaining teeth of the first carriage and the second carriage, respectively, or on equivalent abutment surfaces that hold the carriages in the retracted position, thus preventing the springs from extending.

By dragging the loading buttons to the retracted position, the healthcare professional loads the gun, i.e. brings both the first and the second carriages to the retracted position. By pressing one of the buttons, the healthcare professional releases both carriages, which spring to the forward position under the action of the respective springs. The first carriage completes its travel toward the respective forward position with a minimum delay compared to the time necessary for the second carriage to complete the respective forward travel. The sequential sliding of the cannula with respect to the stylet allows the above-mentioned guillotine-like technique to be implemented.

Once the healthcare professional has inserted the needle into the patient's body and has pressed a button, the pointed end of the stylet comes out of the cannula and the tissue to be sampled is positioned in the collecting hollow. The subsequent sliding of the cannula on the stylet causes the separation of the tissue sample, which remains trapped in the chamber defined by the collecting hollow and the same cannula. In practice, the sharp surfaces of the stylet and cannula cooperate like a guillotine to cut the tissue and hold it in the needle, which can then be removed from the patient's body with the sample contained therein.

The sequential movement of the stylet and cannula is generally achieved by positioning the first carriage slightly retracted compared to the second carriage. The two carriages have identical travels. This feature precisely makes the operation of the gun automatic.

The term automatic therefore refers to the fact that it is not the healthcare professional who operates the stylet and the cannula separately, but these two components move automatically and in due times after the second button is triggered by the healthcare professional.

However, guns are known in which the automatism just described is optional, meaning that the healthcare professional is capable of selectively operating the cannula and the stylet, at the desired times. In this case, we are talking about semi-automatic guns: the stylet is always operated first but the cannula moves forward only when the healthcare professional triggers the respective button, i.e. on command.

The return travel of the carriages to the respective retracted positions allows the springs to be reloaded for a new use. The healthcare professional manually pulls the first button and then on the second button to reload the springs and bring the carriages to the initial retracted position. Before completing the return travel, the healthcare professional ensures that the stylet remains uncovered from the cannula long enough to extract the tissue sample taken from the patient's body from the needle.

At this point, the gun can be used for other sampling on the same patient and then either be replaced or supplied with a new sterile needle for sampling on other patients.

Examples of guns for biopsy are described in KR 20130079788, DE 102011014722, DE 102011014721, DE 102008038414.

US 7,153,275 describes an automatic gun for biopsies, in which the first and second carriages are co-axial and are arranged in front of each other on the longitudinal axis, according to a configuration that can be defined "in-line" The gun comprises a single button for loading the carriages, which is positioned on top of the gun's body, and a triggering button positioned at the rear on the gun's body and adapted to be pressed by the thumb.

US 5,284,156 also describes an automatic gun for biopsy in which the first carriage and the second carriage are configured in line, i.e. they are coaxial, aligned on the longitudinal axis along which the needle extends.

A similar solution is described in US 4,944,308.

US 5,036,860 describes a solution in which one of the carriages is guided into the gun's body in inner guides that have curves at end stops, such as e.g. denoted by reference 124b in the figures.

US 2003/0073929 describes a gun in which the loading of the carriages is provided by telescopically inserting one half of the gun's body into the other half. A triggering button is provided at the rear to release the carriages and allow them to slide forward.

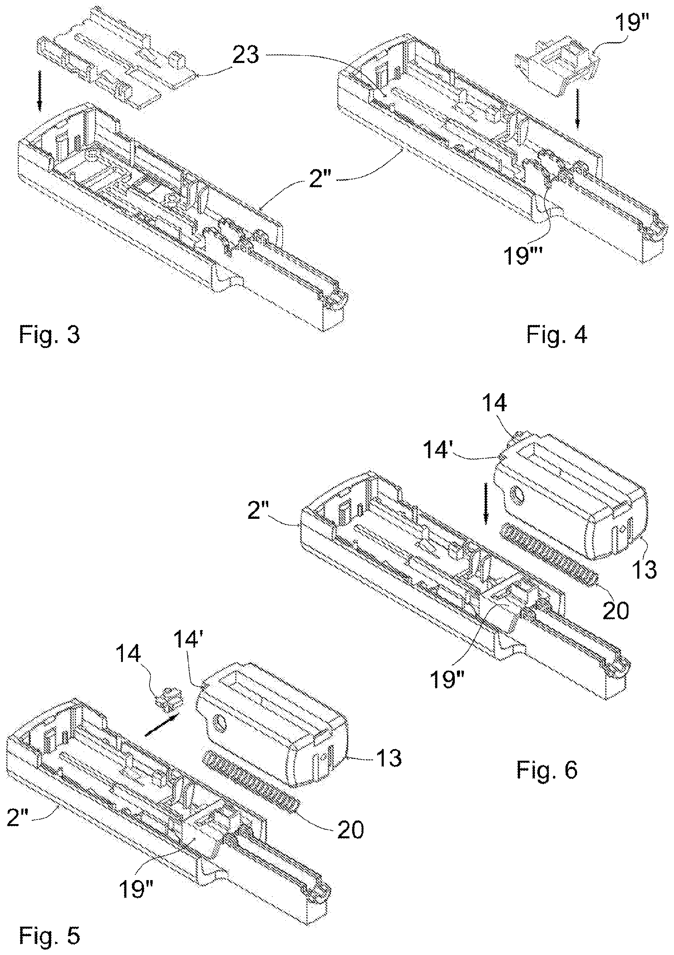

US 5,243,994 describes a gun for biopsy in which the loading of the carriages is operated by the user by means of a cocking member 56 (fig. 3; col. 4, last paragraph) sliding on the gun's body. The cocking member 56 comprises a drive tongue 56b intended to engage the drive member 42. A lever arm mechanism 58b controls a ratchet wheel 66 which acts on a kinematic chain for locking the sliding of the drive members.

US 2008/287825 describes a gun for biopsies, equipped with in-line carriages. The loading button of the carriages is on the top part of the box-shaped body and the firing button is on a side surface of the box-shaped body.

US 2008/287825 describes a gun for biopsy in which the carriages are guided inside the box-shaped body by special guides formed in the inner surface of the body.

US 2005/124914 describes a gun for biopsy in which the stylet rotates with respect to the cannula.

WO 2014/081812 describes a gun for biopsy comprising a single thrust button for loading carriages. The thrust button has two teeth, a first tooth for engaging with the first carriage and a second tooth for engaging with the second carriage. The gun also comprises a gear having the function to facilitate the gun loading in two steps, each step for loading one of the two carriages.

DE 102008038413 describes a gun for biopsy in which the carriages are arranged not in the in-line configuration but are arranged in side-by-side configuration, i.e. they are parallel. The single loading button is equipped with an elastic portion to carry out the loading of the first carriage and elastically deform to engage with the second carriage and allow it to be loaded.

US 2009/299221 describes another solution with side-by-side carriages. In this gun for biopsy, the loading of the carriages takes place by means of a handle present at the rear portion of the box-shaped body, which can be grasped and pulled by the operator to retract both carriages at the same time. The loading element is provided with a flexible selector denoted by reference 136 in the figures, which moves alternately to the right and left of the longitudinal axis, precisely flexing in an elastic manner, to allow selective loading of the cannula carriage and stylet carriage.

A similar solution is described in US 2015/148704.

WO 2017/021826 describes an automatic gun for biopsies, which is equipped with two carriages in parallel, side-by-side configuration, and a single loading button. The carriages are guided by guides inside the box-shaped body and the end stops correspond to curves of the guides.

The manufacturers of guns for biopsy are constantly striving to streamline the structure of guns, for making them easier to build, assemble and use.

Most components of guns for biopsy are made of a plastic polymer by injection moulding. That said, therefore, it is desirable to be able to minimise the number of components and streamline the shape of the necessary components as much as possible. Complicated component geometries, with abutment surfaces, teeth and undercuts, negatively affect production costs and often make the assembly complicated. Currently, one worker takes about 8-10 minutes to complete the assembly of a traditional gun for biopsy. Considering that the market imposes low costs, the time required for the assembly must be as short as possible.

Despite the complicated geometry of the components, in traditional guns for biopsy, the movement of the carriages in both directions is often poorly smooth, sometimes even jerky. In this sense, it is desirable to make guns characterised by greater smoothness of the carriages and by the most flowing possible operation of the carriages and, therefore, of the needle.

### Summary of the invention

Object of the present invention is thus to provide an automatic or semi-automatic gun for biopsies that is simple in structure, economical and functional for the healthcare professional, compared to known solutions, with a limited number of components, and wherein the components are simple to mould and assemble quickly.

Therefore, the present invention concerns a gun for biopsies according to claim 1.

The gun comprises a body that can be held by a healthcare professional, e.g. a box-shaped body having the shape of a grip, and a first carriage and a second carriage housed in the body. There are also elastic means combined with the first carriage and the second carriage which, as will be explained further ahead, have the function of constantly exerting a thrust on the respective carriage in order to move it, a gun loading mechanism, at least one triggering or firing button, and a needle that extends in a cantilevered way from the body along a longitudinal axis.

The needle comprises a stylet constrained to the first carriage and a cannula constrained to the second carriage. The stylet has a pointed end at which there is a hollow for collecting a tissue sample. The stylet is housed in the cannula, which can slide therein, in such a way that the collecting hollow can come out of the cannula and retract therein with a guillotine effect, which allows the tissue sample to be cut.

In order to achieve the guillotine effect just described above, both the cannula and the stylet must move on the longitudinal axis between a retracted position and an extended position. For this reason, the first carriage is movable in the body in a longitudinal direction between a forward position, at which the stylet is completely extended from the body and comes out at least partially of the cannula, and a retracted position, at which the stylet is partially inserted in the body and the collecting hollow is in the cannula. The expression "completely extended from the body" should be understood as meaning that the stylet is in an end stop and maximum extension position outside of the gun's body, not necessarily that the entire stylet extends out of the gun's body. In turn, the second carriage is movable in the body in a longitudinal direction between a forward position, at which the cannula is completely extended from the body, and a retracted position, at which the cannula is partially inserted in the body.

The elastic means mentioned above constantly exert a thrust on the first carriage and the second carriage in the direction of the respective forward position; preferably, the elastic means are coil springs inserted in the gun's body, between a body wall and the respective carriage, and are preloaded.

The gun loading mechanism comprises a thrust element of both the first and the second carriages. The thrust mechanism is constrained to the body and is movable by the operator with respect to the body, in a longitudinal direction, i.e. along the longitudinal axis or parallel to such axis, between an inactive position and a loading position corresponding to at least one of the first carriage and the second carriage in the retracted position, which corresponds to the loading of the carriage having taken place.

The loading mechanism comprises a slider mounted on the thrust element and slidably movable with respect to the same thrust element orthogonally to the longitudinal axis, in both directions. The function of the slider is to allow the selective loading of one of the two carriages when the operator acts on the thrust mechanism. In fact, the body of the gun comprises a longitudinal guide branched into a first branch and a second branch; the slider selectively engages the first or second branch, in response to the retraction of the thrust element into the loading position, depending on whether the loading of the second carriage or the first carriage is carried out, respectively.

Advantageously, the selection is automatic, meaning that the slider sequentially engages the first branch and the second branch automatically when the operator drags the thrust element backwards.

This solution, based on the use of the slider sliding on the thrust element and the branched guide, allows the gun to be made with a loading mechanism that is extremely simple in structure, geometry and movements. With a single thrust element, in fact, the operator can first load the second carriage, with a first longitudinal movement of the thrust element, and immediately after loading the first carriage, returning the thrust element to the initial position and imparting a second longitudinal movement to the same thrust element. This operation does not require components of complex geometry, which are difficult to mould; on the contrary, it can be achieved with a limited number of components, with a relatively simple geometry, easy to mould and assemble at low cost. In addition, this configuration allows to avoid using an elastic hinge, i.e. of fins or other flexible elements whose behaviour over time is unpredictable due to the relaxation of the plastic material of which the flexible elements are made.

The use of the sliding slider also ensures consistency in the resistance offered by the gun to loading, i.e. at the moment of the loading element by the operator. In fact, the slider always encounters the same friction in the respective seat on the loading element, unlike a flexible element, whose flexural strength often changes over time due to the relaxation of the material constituting it.

Generally, the use of the sliding slider allows to minimise the force necessary to load the gun, which provides greater confidence to the operator and a perception of gun quality.

Using an analogy, the slider and the branched guide operate similarly to a railway switch, which allows to selectively select the railway branch on which a train can, from time to time, advance.

As set forth above, advantageously, the slider sequentially engages the two branches of the guide autonomously, due to the geometry of the guide.

The configuration described also allows a loading element to be used, which is positioned on the front of the gun's body, i.e. around the needle, with the result that the operation of the loading element can be achieved even with one hand.

In the preferred embodiment, the first carriage and the second carriage are arranged side by side in the gun's body, even more preferably, they are in shoulder-to-shoulder abutment and move parallel to the longitudinal axis, in both directions.

As mentioned above, the loading of the gun is sequential: the slider engages the first branch of the longitudinal guide in response to a first retraction of the thrust element into the loading position; at this point, the second carriage is brought to retracted position and held therein, and the thrust element may return to the inactive initial position in order to allow the loading of the first carriage, during which the slider engages the second branch of the longitudinal guide in response to a second retraction of the thrust element to the loading position. The first carriage is also held in the retracted position. Thus, following a double triggering of the thrust element by the operator, the sequential loading of the second carriage and the first carriage is achieved and the gun is then ready to fire.

In more detail, the retracted position of the thrust element, with the slider engaged in the first branch of the longitudinal guide and, preferably, at the end stop, corresponds to the second carriage in the retracted position, which is the position from which it begins to move when fired.

Similarly, the retracted position of the thrust element, with the slider engaged in the second branch of the longitudinal guide, corresponds to the first carriage in the retracted position.

In practice, the operator presses the thrust element twice consecutively in order to obtain the sequential loading of the two carriages and, therefore, bring the gun into the ready-to-fire configuration.

Preferably, at least one of the two carriages is provided with a portion shaped to cooperate with the longitudinal guide in guiding the slider. In other words, the longitudinal guide is partially defined by gun's body surfaces and partially by surfaces of a carriage or both carriages.

In the preferred embodiment, at least one of the first carriage and the second carriage is shaped with a wedge-shaped portion that, together with the longitudinal guide, defines the path of the slider.

More in detail, the longitudinal guide comprises a distal end positioned closer to the needle than the first branch and the second branch that precisely depart from the distal end. The distal end of the longitudinal guide is triangular and the vertex is occupied by the slider when the thrust element is in the respective inactive position. The slider slidingly and selectively engages either side of the longitudinal guide at the distal end when the thrust element is pushed by the operator toward the loading position, depending on which side the slider engages, whether left or right, the slider continues its movement in either the first branch or the second branch of the guide, in order to allow for what has been referred to above as selective loading of the carriages.

Preferably, the angle at the vertex of the distal end of the longitudinal guide is asymmetrical with respect to the longitudinal axis, meaning that the two sides of the distal end, right and left, form different angles with the median plane of the gun passing through the longitudinal axis.

Preferably, the two sides, right and left, of the vertex of the distal end of the longitudinal guide are different in length.

In practice, in the preferred embodiment, the longitudinal guide has a first end with a vertex from which the two branches described above depart and the vertex is asymmetrical with respect to the median plane of the gun, which passes through the longitudinal axis.

In an embodiment, the thrust element is a single element functionally combined with both the first carriage and the second carriage, to cause its retraction, and is constrained to the body telescopically, in an intermediate position between the body and the needle.

An embodiment of the gun offers the operator the possibility to set the travel of the carriages and, therefore, the travel of the stylet and cannula, between at least two values, e.g. 18 mm and 25 mm.

Preferably, the loading mechanism comprises a selector with a function of stopping the carriages; the selector is interposed between the first carriage and the second carriage on one side and the body on the other side. The selector is movable by the operator with respect to the gun's body between a first position, corresponding to a reduced travel of the first carriage and the second carriage, and a second position corresponding to an extended travel of the first carriage and the second carriage. In practice, the selector functions as a spacer functionally interposed between the carriages and the gun's body, and has two portions of different thickness, a thicker portion and a thinner portion: the displacement of the selector results in only one of the two portions acting as end stops of the carriages.

The thrust element is preferably counteracted by its elastic element, e.g. a coil spring, which constantly exerts a thrust to return the thrust element to its inactive position. When the operator uses the thrust element to load the gun, i.e. to retract the carriages, it must overcome the force of the elastic element just described.

The gun can be made with a single firing button or multiple firing buttons in different positions to improve the ergonomics for the operator or to allow the operation in two modes, automatic and semi-automatic.

In an embodiment, the gun comprises a first triggering or firing button, whose pressure by the operator causes both carriages to automatically move forward to the forward position, with the necessary timeline to ensure the guillotine effect, and a second triggering or firing button, whose pressure by the operator causes only the first carriage to automatically move forward to the forward position. In this case, i.e. when the operator acts on the second button, the stylet moves forward first, pushed by the first carriage, but the cannula and the second carriage remain stationary; the forward movement of the cannula and the second carriage is dependent on the operator pressing the first firing button. Thus, in semi-automatic mode, the operator manually regulates the duration of the firing sequence.

When they are in the retracted position, the two carriages are held by special means of the gun's body which prevent inadvertent forward movement, i.e. the forward movement not dependent on pressing a firing button.

Preferably, the gun comprises at least two flexible and elastic fins which extend in a cantilevered way from the inner surface of the body or from a plate housed in the body. Each fin configures an undercut with one of the two carriages to hold it in the retracted position, by overcoming the thrust of the elastic means combined with the two carriages. For example, each carriage comprises a tooth engaged by the respective elastic fin. When the operator presses the firing button, or the buttons if there are more, it causes the bending of one or both fins, resulting in the carriages being released and being able to be translated to the respective forward position by sliding into their respective seats.

Ultimately, therefore, the solution proposed is a gun that is simple in structure, can be made with a small number of low-cost components by polymer moulding, can be assembled quickly, in less time than common guns for biopsy available today, easy to use but at the same time it allows both automatic and semi-automatic use and the regulation of the stylet and the cannula travels.

### Brief list of the figures

Further characteristics and advantages of the invention will become clearer in the review of the following detailed description of a preferred, although not exclusive, embodiment illustrated by way of example and without limitations with the aid of the accompanying drawings, in which:
- Figure 1 is a perspective view of a fully assembled automatic gun for biopsies according to the present invention;
- Figure 2 is a perspective and exploded view of the gun shown in figure 1;
- Figures 3-12 are perspective views showing the assembling sequence of the gun shown in figure 1;
- Figures 13-24 are top views or plan sectional views or vertical sectional views showing the loading sequence of the gun shown in figure 1;
- Figures 25-31 are top views or plan sectional views or vertical sectional views showing the firing sequence of the gun shown in figure 1, for a first length of the needle travel;
- Figures 32-38 are top views or plan sectional views or vertical sectional views showing the firing sequence of the gun shown in figure 1, for a second length of the needle travel;
- Figure 39 is a plan sectional view of the gun shown in figure 1, showing the second end stop of the needle.

### Detailed description of the invention

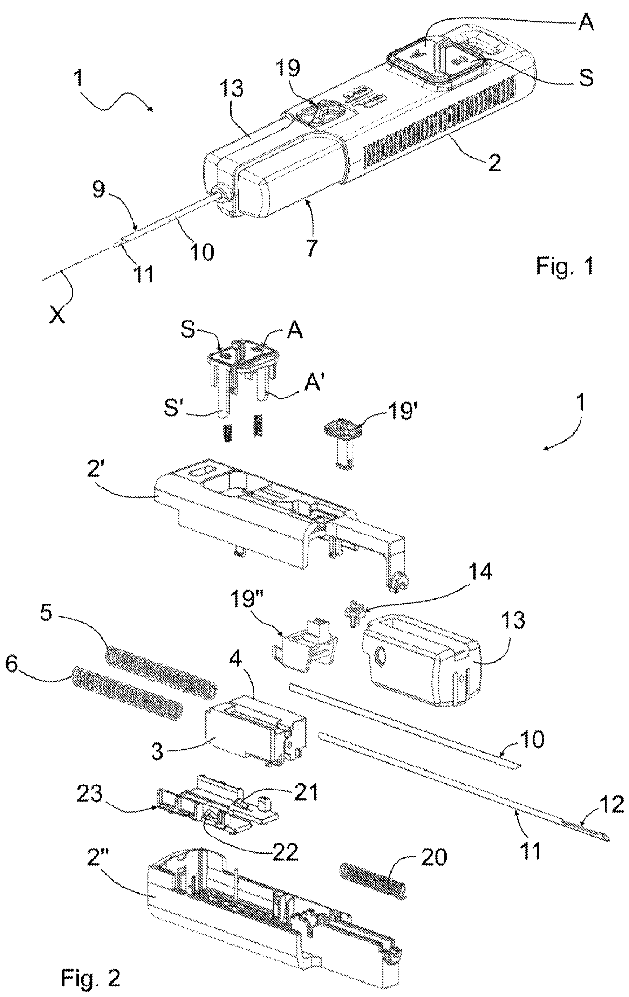

Figure 1 is a perspective view of an automatic gun 1 for biopsies according to the present invention, which is provided with a box-shaped body 2, a loading mechanism 7 and a needle 9 intended to be inserted into a patient's body for taking a tissue sample. The needle 9 in turn consists of a stylet 11 slidably and coaxially inserted into a cannula 10. Both the stylet 11 and the cannula 10 are movable on the longitudinal axis X in order to achieve the guillotine effect described above.

The loading mechanism 7 comprises at least one thrust element 13 and, preferably, as shown in the example in the figures, a single thrust element 13. In particular, in the gun 1, the thrust element 13 is positioned between the needle 9 and the body 2 and is telescopic with respect to the body 2; in other words, the thrust element 13 can be pressed by the operator and retracted at least partially into the body 2 of the gun, by exerting a longitudinal thrust to carry out the loading, as will be described hereunder.

On the body 2, two firing buttons A and S can also be seen, which can be accessed from the top, the button A to perform the tissue sampling in completely automatic mode and the button S to perform the tissue sampling in completely semi-automatic mode, as will be described further hereunder.

A selector 19 is present at the upper surface of the gun 1; the selector is movable between two positions, right and left, in order to allow the operator to select a corresponding extension of the needle 9 when fired. In the example shown in the figures, the gun 1 is configurable through the selector 19 in order to fire at 18 mm or to fire at 25 mm; in the first case, the stylet 11 and the cannula 10 move suddenly forward 18 mm and, in the second case, 25 mm.

Figure 2 is a perspective and exploded view of the gun 1 in which it is possible to observe all the components, even the inner ones.

The body 2 is formed by two complementary shells, the upper shell 2' and the lower shell 2'.

The lower shell 2" houses a plate 23 equipped with flexible fins 21 and 22 which define an undercut or a retaining tooth of the carriages 3 and 4, whose function will be described hereunder.

The cannula 10 and the stylet 11 are clearly visible; the latter is provided with a collecting hollow 12 for collecting the patient's tissues. By longitudinally sliding inside the cannula 10, the stylet 11 together with the cannula 10 allows a piece of tissue to be cut and held in the hollow 12 for the extraction from the patient's body and the subsequent collection as a histological sample.

The gun 1 further comprises a first carriage 3, to which the stylet 11 is fastened in a stable manner, and a second carriage 4, to which the cannula 10 is fastened in a stable manner. The two carriages 3, 4 have the same longitudinal extent and are assembled side by side, i.e. shoulder to shoulder, being able to slide with respect to each other. The task of the carriages 3 and 4 is to thrust, respectively, the stylet 11 and the cannula 10 on the longitudinal axis X in the selected timeline, depending on whether the operator presses the button A or the button S.

Generally, the carriages 3 and 4 are selectively and sequentially movable in the body 1 between a forward position and a retracted position, which respectively correspond to the stylet 11 and the cannula 10 completely extended from the body 2 and partially retracted in the body 2.

In the body 1, elastic means 5, 6, which are used to counter the movements of the carriages 3 and 4 and, in particular, are used to constantly exert a longitudinal thrust on the carriages 3 and 4 to bring them toward the respective forward position, are housed. In the example shown in the figures, the elastic means 5, 6 are coil springs interposed between the carriages 3 and 4 and the rear surface of the body 2, in particular the lower shell 2".

As can be seen, the selector 19 consists of a lever 19' which is accessible from the top of the body 2 and operable by the operator with a finger and a lower element 19" which, when the gun 1 is assembled, is connected to the lever 19'. As will be described later, the selector 19 can be translated in both directions along a direction orthogonal to the longitudinal axis X.

The object of the selector 19 is to selectively change the travel of the needle 9, between a first firing length and a second firing length. In the example shown in the figures, the two firing lengths are 18 mm and 25 mm, respectively. Thus, by means of the selector 19, the operator can set one of the two firing lengths, i.e. selectively limits the travel of the carriages 3 and 4 because, depending on the position of the selector 19, correspondingly changes the end stop surface against which the carriages 3 and 4 are brought to abutment when the gun 1 is operated.

In the body 2, an elastic element 20 is also housed for counteracting the thrust element 13: in the example shown in the figures, it is a coil spring 20 functionally interposed between the thrust element 13 and an abutment surface of the lower shell 2".

The thrust element 13 is constrained to the body 2 of the gun 1 in a telescopic manner, i.e. it is movable in both directions in the longitudinal direction, between a completely retracted position and a completely extended position (which can be seen in figure 1). The thrust element 13 is intended to be pressed by the operator, with the fingers of one hand, in order to carry out the loading of the gun 1, i.e. to bring both carriages 3 and 4 to the retracted position. The spring 20 has the task of returning the thrust element 13 to the completely extended position after each loading.

The gun 1 also comprises a slider 14 mounted on the thrust element 13 and movable with respect thereto orthogonally to the longitudinal axis X, in both directions. As will be explained later, the slider 14 has the task of transferring the thrust exerted by the thrust element 13 selectively to one of the two carriages 3 or 4 at a time. In other words, each retraction of the thrust element 13 imparted by the operator results in the retraction of only one of the two carriages 3 and 4 by virtue of the position taken by the slider 14 with respect to the thrust element 13, with the result that, in order to bring both carriages 3 and 4 into the retracted position, thus completing the loading of the gun 1, the operator shall retract the thrust element 13 twice consecutively. A first time to retract the second carriage 4 and, in this case, the slider 14 aligns with the second carriage 4 and a second time to retract the first carriage 3 and, in this case, the slider 14 aligns with the first carriage 3.

The slider 14 is intended to move as guided within a longitudinal guide 15 which cannot be seen in figures 1 and 2. It is revealed herein that the longitudinal guide 15 is branched into a first branch 15' and a second branch 15"; the slider 14 selectively engages the first branch 15' or the second branch 15', in response to the retraction of the thrust element 13, to carry out the loading of the first carriage 3 and the second carriage 4, respectively.

Figures 3-12 are a perspective view of the gun 1 during the respective assembly, i.e. during the mounting of the different components.

The diagram of assembly initially provides for the positioning of the plate 23 in the shell 2", directly on the bottom of the shell 2", into which it fits (figure 3). Subsequently, the lower element 19" of the selector 19 is then placed on the shell 2", in particular in a chase 19"' which acts as a transverse guide orthogonal to the longitudinal axis X, in which chase 19‴ the element 19" is sliding in both directions (figure 4). The thrust element 13 with the respective counteracting spring 20 is then positioned, taking care to insert the slider 14 on a corresponding transverse guide 14' present on the thrust element 13 from the side facing the carriages 3 and 4, i.e. the side opposite the needle 9 (figures 5-6).

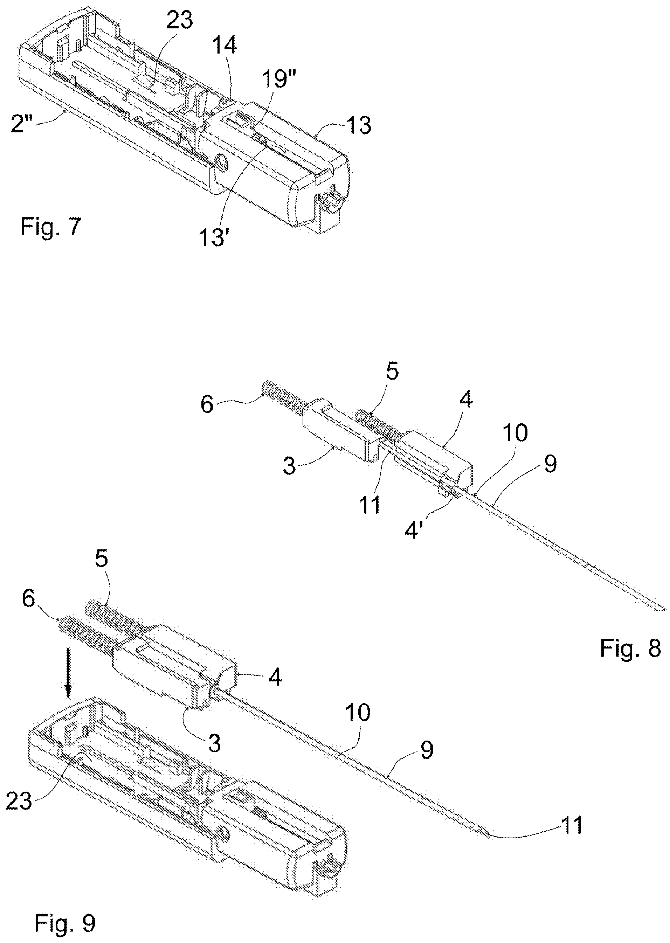

As shown in figure 7, the assembling provides that the lower element 19" of the selector 19 engages the longitudinal guide 13' of the thrust element 13. This configuration allows the retraction of the thrust element 13 in the body 2 of the gun 1 and, at the same time, allows the selector 19 to be moved transversely in the guide 13', since the guide is sufficiently large.

Subsequently, as shown in figures 8-10, the carriages 3 and 4 are assembled. The first carriage 3 and the second carriage 4 are placed side by side, as shown in figure 8. It should be noted that the second carriage 4 is provided with a front hole 4' through which the stylet 11 fastened to the first carriage 3 is slidably inserted. The cannula 10 is fastened to the second carriage 4. The two side-by-side carriages 3 and 4 and the respective springs 6, 5 are inserted into the body 2, with the springs 5, 6 preloaded between the bottom wall of the body 2 and the carriages 3, 4. The needle 9 is complete. The carriages 3, 4 inserted into the shell 2" slide on the plate 23.

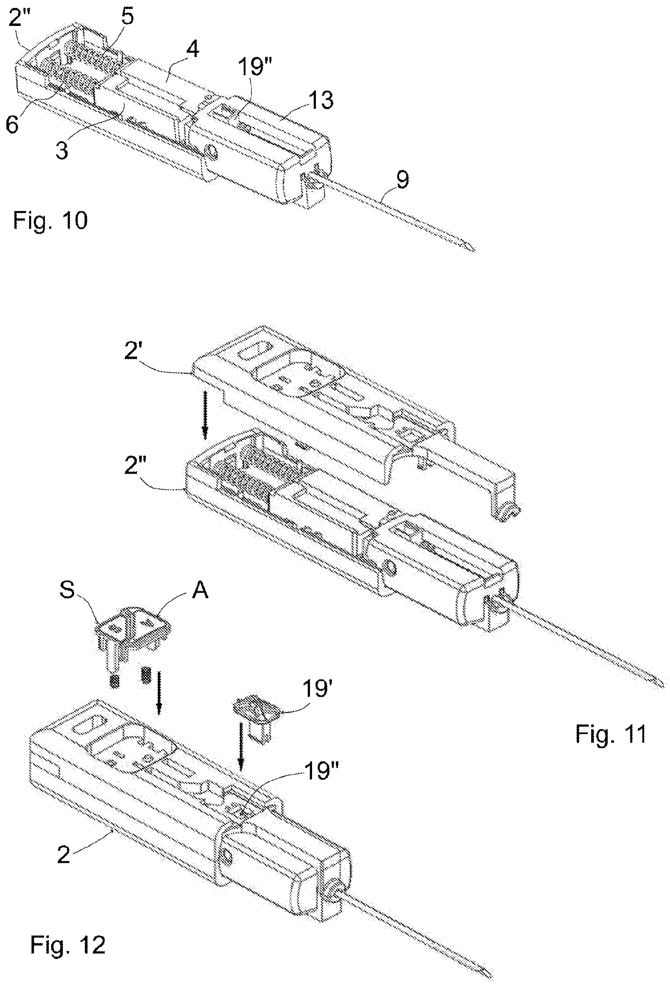

At this point the body 2 is closed, by joining the shells 2' and 2" (figure 11), and the two firing buttons A and S and the lever 19' of the selector 19 (figure 12) are installed.

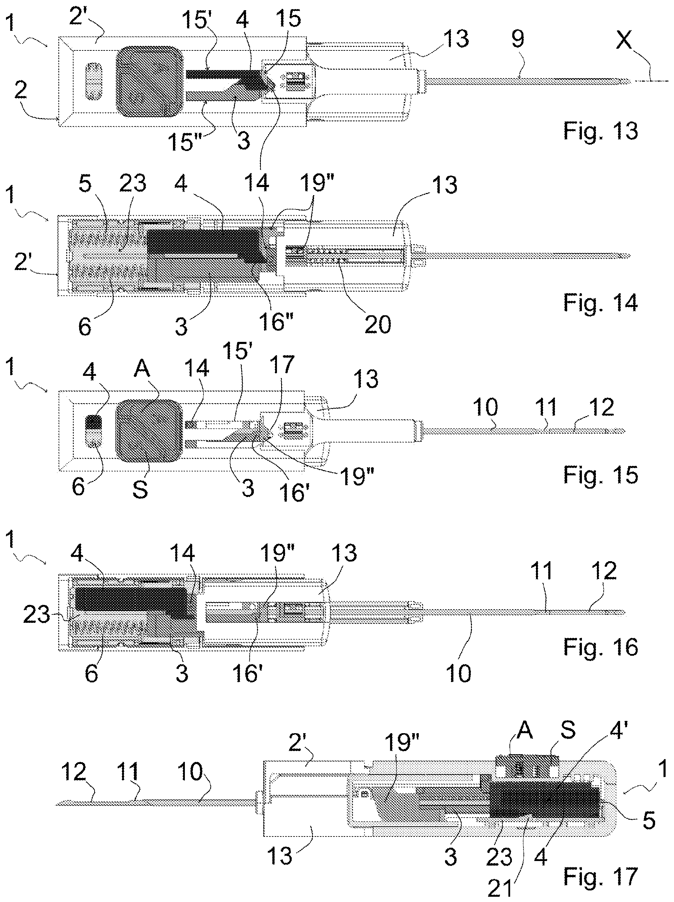

Figures 13 to 24 show the loading sequence of the gun 1.

In particular, figure 13 is a plan and top schematic view of the gun 1 in the initial, unloaded configuration. A longitudinal guide 15 formed in the body 2 can be seen, and in particular in the upper shell 2', which is branched into a first branch 15' and a second branch 15". The first branch 15' is aligned longitudinally with the second carriage 4 and the second branch 15" is aligned longitudinally with the first carriage 3.

The slider 14 selectively engages the first branch 15' or the second branch 15", in response to the retraction of the thrust element 13 to the loading position. In other words, the slider 14 follows the first branch 15' of the guide 15 to allow the loading of the second carriage 4, when the thrust element 13 is pressed for the first time and then follows the second branch 15" of the guide 15 to allow the loading of the first carriage 3, when the thrust element 13 is pressed a second time.

In figure 13, the carriages 3 and 4 are both in the forward position, in abutment against the selector 19, and the thrust element 13 is at rest in the position completely extended from the body 2. The slider 14 is at the apex of the distal end 17 (figure 15) of the guide 15 and is mounted on the thrust element 13 and movable with respect thereto orthogonally to the longitudinal axis X, in both directions, on the guide 14' which can be seen in figures 5 and 6.

Figure 14 is a view of the gun 1 in the same configuration shown in figure 13 but without the upper shell 2', to better show the position of the carriages 3, 4 and the slider 14. The guide 15 cannot therefore be seen but the carriages 3 and 4 can be seen and are held in the forward position by the springs 5 and 6. As can be seen, the second carriage 4 is shaped with a wedge-shaped end portion 16" which, together with the longitudinal guide 15, defines the path of the slider 14, particularly in the first instants of its movement, in order to help route it correctly into the first branch 15'. In particular, by virtue of the cooperation between the guide 15 and the wedge-shaped end portion 16" of the second carriage 4, when the thrust element 13 is pressed by the operator and brought to the retracted position, the first carriage in chronological order that is loaded, i.e. brought to the retracted position, is precisely the second carriage 4.

Figure 15 shows the gun 1 in a top plan view, with the upper shell 2' mounted and, therefore, with the guide 15 visible and the second carriage 4 in the retracted position. As can be seen, the slider 14 has completely travelled the first branch 15' of the guide 15, thus reaching the end stop. The first carriage 3 is, on the contrary, still in the forward position, in abutment against the portion 19" of the selector 19 and, in fact, the coil spring 6 is visible alongside the second carriage 4. In this configuration, with the first carriage 3 in the forward position and the second carriage 4 loaded in the retracted position, the cannula 10 is retracted and the collecting hollow 12 of the stylet 11 is exposed.

Figure 16 shows the same view of the gun 1 as figure 15 but with the upper shell 2' omitted.

Figure 17 is a sectional view of the gun 1, which is considered on a vertical plane passing through the longitudinal axis X, in the same configuration shown in figures 15 and 16, i.e. with the second carriage 4 being retracted and the first carriage 3 being in the forward position. As can be seen, the second carriage 4 is held in a retracted position, against the thrust exerted by the spring 5, by a flexible fin 21 of the plate 23, which engages an undercut 4' of the second carriage 4 at its lower surface. As will be explained hereunder, when the operator presses the firing button, the pressure causes the flexible fin 21 to bend in the direction of the plate 23, i.e. it flattens the fin, resulting in the disengagement of the second carriage 4 which will be free to move forward as a result of the thrust exerted by the spring 5.

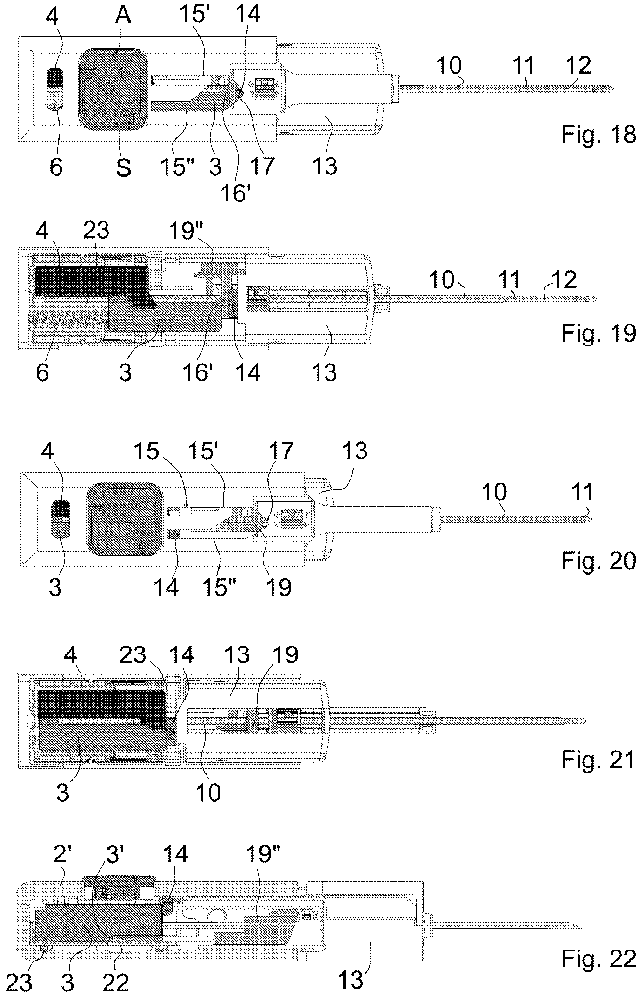

Figure 18 is a top plan view of the gun 1 with the second carriage 4 in the retracted position, as shown in figures 15-17, but with the slider 14 again positioned at the apex of the distal end 17 of guide 15: after the operator first presses the thrust element 13, in fact, the same thrust element 13 returns to the forward, initial inactive position as a result of the thrust exerted by the coil spring 20 (figure 6). Since the slider 14 is constrained to the thrust element 13 (please note that it is sliding in a guide of the thrust element 13 orthogonal to the longitudinal axis), it is dragged back along the first branch 15', i.e. it travels along it in the direction opposite the previous one, in order to be brought to the initial position corresponding to that of figures 13 and 14. In this position, the slider 14 is ready for the second triggering of the thrust element 13. As can be seen, the first carriage 3 also is provided with a wedge-shaped end portion 16' whose function is to guide, together with the guide 15, the slider 14 and, in this case, to route it into the second branch 15' during the second pressing of the thrust element 13.

Figure 19 shows the same view of the gun 1 as figure 18 but with the upper shell 2' omitted. Since the second carriage 4 is in the retracted position and does not intervene in the routing of the slider 14, a new pressing of the thrust element 13 causes the slider 14 to be diverted from the wedge-shaped end portion 16' of the first carriage 3 in order to enter the second branch 15" of the guide 15.

This condition is shown in figure 20, which shows the gun 1 in a top plan view, with the upper shell 2' mounted and, therefore, with the guide 15 visible and both carriages 3 and 4 in the retracted position. As can be seen, the slider 14 has completely travelled the second branch 15" of the guide 15, thus reaching the end stop. In this configuration, with both carriages 3 and 4 in the retracted position, both the cannula 10 and the stylet 11 are retracted and the collecting hollow 12 of the stylet 11 is not exposed but is inside the cannula 10.

Figure 21 shows the same view of the gun 1 as figure 20 but with the upper shell 2' omitted. As can be seen, the carriages 3 and 4 remain parallel and side by side at all times, whether they are both in the forward or retracted position, or they are staggered with one carriage in the forward position and the other in the retracted position.

Figure 22 is a sectional view of the gun 1, which is considered on a vertical plane passing through the longitudinal axis X, in the same configuration shown in figures 20 and 21, i.e. with both carriages 3 and 4 being retracted: this is the configuration of the gun 1 ready for firing. As can be seen, the first carriage 3 is also held in retracted position, against the thrust exerted by the spring 6, by a flexible fin 22 of the plate 23, which engages an undercut 3' of the first carriage 3 at its lower surface. As will be explained later, when the operator presses the firing button, the pressure causes the flexible fin 22 to bend in the direction of the plate 23, i.e. it flattens the fin 22, resulting in the disengagement of the first carriage 3 which will be free to move forward as a result of the thrust exerted by the spring 6.

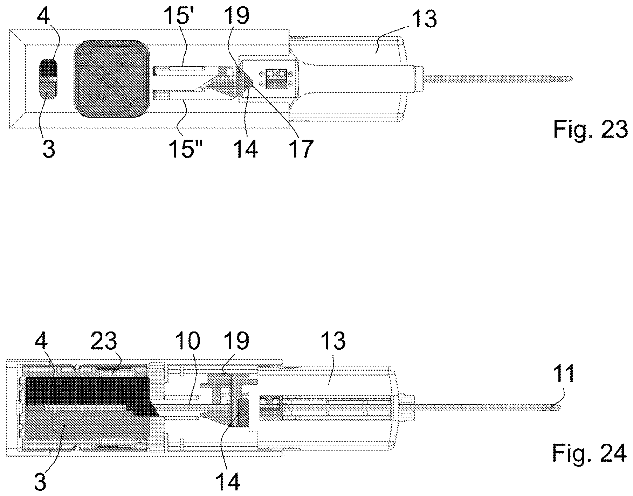

Figure 23 shows the gun 1 in plan view, from the top, with both carriages 3 and 4 in the retracted position, in the configuration of the gun 1 loaded and ready for firing, as shown in figures 20-22, but with the slider 14 back in the initial position, at the apex of the distal end 17 of the guide 15. This is because after the second triggering of the thrust element 13, which is necessary to retract the first carriage 3, the thrust element 13 returns to the forward, initial inactive position as a result of the thrust exerted by the spring 20 and drags the slider 14 with it, which retraces backwards the second branch 15" of the guide 15 and returns to the apex of the distal end 17 of the guide 15.

The gun 1 is ready for firing.

Figure 24 shows the same configuration as figure 23 but with the upper shell 2' removed to see the mutual position of the carriages 3 and 4, the selector 19 and the slider 14, as well as the position of the cannula 10 and the stylet 11.

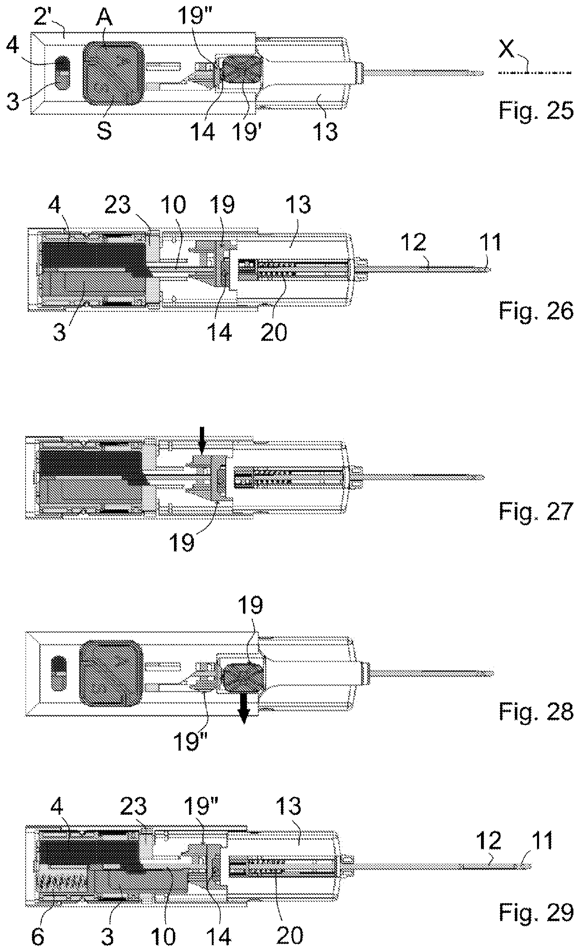

The firing sequence of the gun 1 will now be described, with reference to figures 25-31, for a first firing length corresponding to 18 mm.

In more detail, figures 25-28 show how to use the selector 19 and figures 29-31 show how firing actually takes place.

Figure 25 is a top plan view of the gun 1 ready for firing, with the carriages 3 and 4 both held in the retracted position and the slider 14 in the forward position, the same configuration shown in figures 23-24. The element 19' of the selector 19 is displaced completely to the left with respect to the longitudinal axis X, in a position corresponding to an end stop of 25 mm of the carriages and, therefore, of the needle 9. As will now be described, in order to change the end stop to 18 mm, the operator displaces the selector 19 to the opposite position, i.e. to the left of the longitudinal axis X.

The element 19" of the selector 19 is displaced with respect to the longitudinal axis X together with the element 19' on which the operator acts directly with a finger, e.g. with his thumb.

Figure 26 is a view similar to the view of figure 25 but with the upper shell 2' omitted. The carriages 3 and 4 are both retracted, side by side and held by the fins 21, 22 of the plate 23; the cannula 10 and the stylet 11 are retracted and the collecting hollow 12 is inside the cannula 10. The selector 19 is still in the position corresponding to an end stop of 25 mm.

At this point, as shown in figures 27 and 28, the operator who wishes to reduce the end stop to 18 mm, displaces the selector 19 to the corresponding position, to the right of the longitudinal axis X, and fires.

Figure 29 shows the start of the firing sequence.

The firing can take place in completely automatic mode by pressing the button A, or in semi-automatic mode by pressing the button S.

Assuming the button A is used, its pressure causes the fin 22 to bend, which holds the first carriage 3. As the constraint between the fin 22 and the undercut 3' of the first carriage 3 is no longer present, as shown in figure 29, the first carriage 3 moves suddenly to the forward position, reaching the respective end stop determined by the element 19" of the selector 19. As a result, the stylet 11 partially comes out of the cannula 10 for a length sufficient to expose the collecting hollow 12. The needle 9 is supposed to be inserted into the body of the patient subjected to tissue sampling.

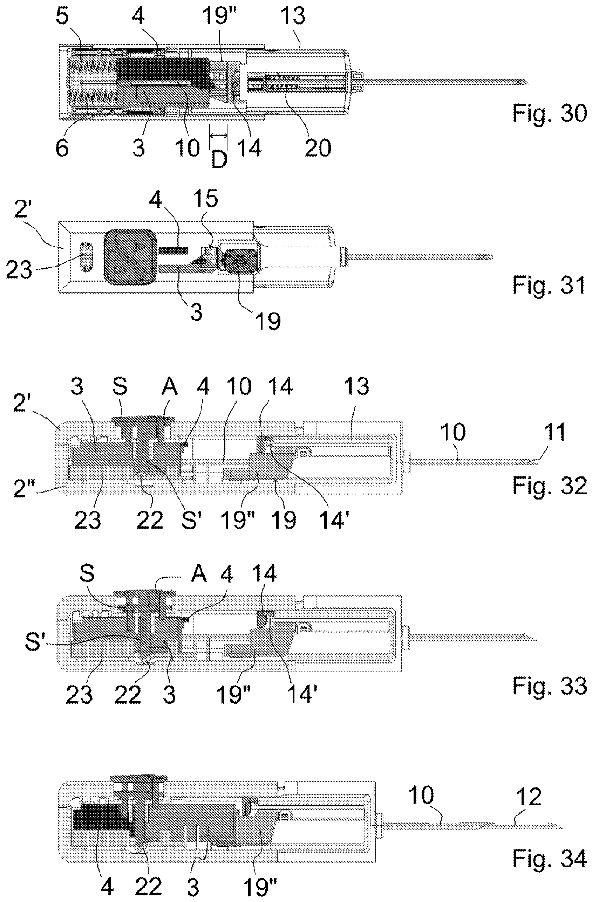

Immediately afterwards, as shown in figure 30, the second carriage 4 also moves forward to bring the cannula 10 to the forward position and to achieve the guillotine effect described above. The release of the second carriage 4 is determined by the fact that the button A is specially shaped to operate first on the fin 22 and then on the fin 21 of the plate 23, which hold the first carriage 3 and the second carriage 4, respectively. Therefore, pressing the button A automatically results in the release of the two carriages 3 and 4 with the necessary timeline: in practice, the second carriage 4 is released forward as soon as the first carriage 3 reaches the end stop in the forward position. The end stop of the second carriage 4 in the forward position is also determined by the element 19" of the selector 19, so that even in the forward position the two carriages 3 and 4 are perfectly aligned and side by side. The cannula 10 slides over the stylet 11 until it covers the collecting hollow and contains the tissue present therein.

Figure 31 shows the gun 1 in the same configuration shown in figure 30, i.e. after it has been fired but with the upper shell 2' visible.

At this point, having collected the tissue from the subject, the operator can extract the needle 9 from the subject.

The firing sequence will now be described again, with reference to figures 32-38, assuming that the operator displaces the selector 19 to the position corresponding to the firing length of 25 mm, i.e. greater than the length of 18 mm pertaining to figures 28-31, and returns it to the position to the left of the longitudinal axis X, as shown in figure 25.

Figures 32-38 are vertical sectional views considered on a vertical plane passing through the longitudinal axis X.

Figure 32 shows the gun 1 charged, with both carriages 3 and 4 in the retracted position and held by the fins 21 and 22 of the plate 23. As can also be seen from a comparison with figure 2, the buttons S and A are provided with corresponding pins S' and A' that extend just toward the plate 23 and have the function of imparting the bending of the fins 21 and 22 in order to achieve the release of the carriages 3 and 4.

When the operator presses the button A, the button S is also lowered because it is dragged by the button A, and this results in both pins A' and S' bending the corresponding fins 21 and 22, with the described timeline, i.e., the pin S' which knocks down the flexible fin 22 before the pin A' knocks down the flexible fin 21. This is due to the fact that the pins A' and S' have different lengths.

When the operator presses the button S, the button S lowers and the pin S' knocks down the fin 22, thus releasing the first carriage 3 but without this affecting the button A and the second carriage 4 which remain stationary.

Thus, in the fully automatic mode, pressing the button A also lowers the button S and the carriages 3 and 4 both move forward suddenly in the sequence described above, whereas in the semi-automatic mode, pressing the button S results in the forward movement of only the first carriage 3 while the second carriage 4 remains stationary and can only move forward after the operator specifically presses the button A.

The difference is that in the semi-automatic mode, the operator decides how long it takes between the forward movement of the first carriage 3 and the forward movement of the second carriage 4.

This circumstance can be easily understood by observing figure 33, which shows the gun 1 with the carriages 3 and 4 still retracted but with the button S pressed: the pin S' has knocked down the flexible fin 22 to free the first carriage 3 and allow it to move forward. As can be seen, the button A and the second carriage 4 remain stationary, with the button A raised and the second carriage 4 retracted.

At this point, the first carriage 3 moves suddenly toward the forward position shown in figure 34. It is well noted that the end stop of the first carriage 3 consists of the element 19" of the selector 19. Thus, by appropriately shaping the selector 19, the length of the firing, i.e. the travel of the stylet 11 and the cannula 10, can be regulated.

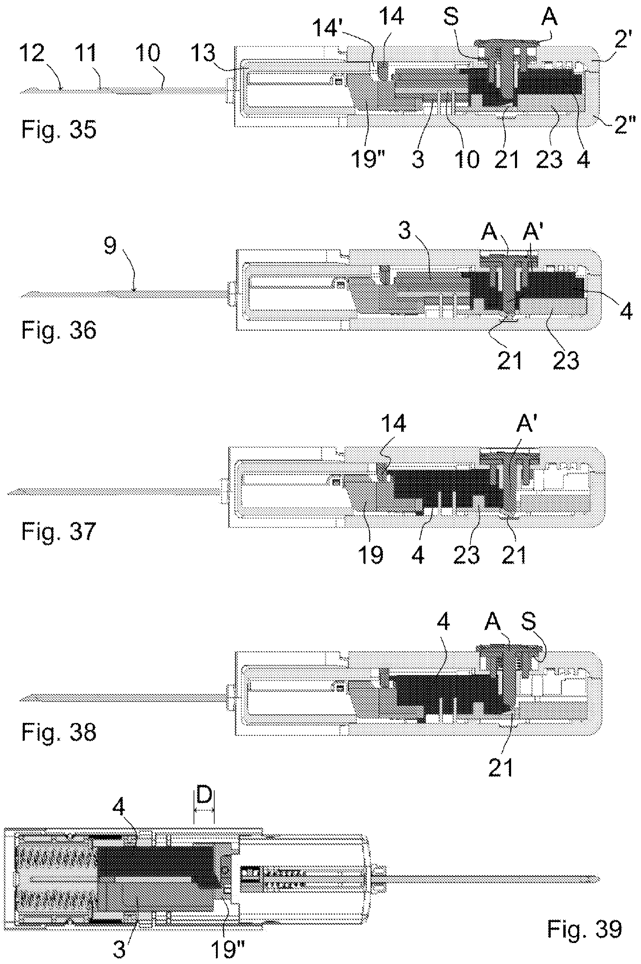

Figure 35 shows the gun 1 in the same configuration shown in figure 34 but from the opposite side. As can be seen, the button A remains raised and the respective pin A' does not act on the fin 21.

Figure 36 shows a subsequent moment of the firing sequence in semi-automatic mode: the operator pressed button A, which resulted in the pin A' knocking down the fin 21 which holds the second carriage 4.

At this point, therefore, as shown in figure 37, the second carriage 4 also moves suddenly to the forward position until it reaches the end stop in abutment against the selector 19, in order to be placed side by side, by aligning with, the first carriage 3. The firing ends.

Figure 38 shows the gun 1 after firing, as in figure 37, but with both the buttons A and S raised again and the fins 21 and 22 raised again.

Figure 39 should be compared with figure 30 to understand how the selector 19 works to change the end stop of the carriages 3 and 4, according to the longitudinal distance D shown precisely in figures 30 and 39. As can be seen, in figure 30 the selector 19 is in a position that offers an early end stop to the carriages 3 and 4, compared to the end stop offered by the same selector 19 in the position of figure 39 (distance D zeroed out).

As can be seen from the figures, the guide 15 is asymmetric, meaning that the triangular vertex 17 forms, with the longitudinal axis X, non-symmetrical angles. This is used to correctly route the slider 14 initially in the first branch 15' and, subsequently, in the second branch 15". In practice, the vertex 17 of the guide 15 is not symmetrical with respect to the longitudinal axis X.

## Claims

1. A gun (1) for biopsies, comprising a body (2) that can be held by an operator, a first carriage (3) and a second carriage (4) housed in the body (2), elastic means (6, 5) combined with the first carriage (3) and the second carriage (4), a loading mechanism (7) of the gun, and at least one button (A, S) for triggering or firing the gun (1), and a needle (9) that extends in a cantilevered way from the body (2) along a longitudinal axis (X),
wherein the needle (9) comprises a stylet (11) constrained to the first carriage (3) and a cannula (10) constrained to the second carriage (4),
wherein the stylet (11) has a pointed end at which there is a collecting hollow (12) for collecting a tissue sample, and
wherein the stylet (11) is housed in the cannula (10), which can slide therein, in such a way that the collecting hollow (12) can come out of the cannula (10) and retract therein with a guillotine effect, and
wherein the first carriage (3) is configured to move in the body (2) in a longitudinal direction between a forward position, at which the stylet (11) is completely extended from the body (2) and comes out at least partially of the cannula (10), and a retracted position, at which the stylet (11) is partially inserted in the body (2) and the collecting hollow (12) is in the cannula (10), and
wherein the second carriage (4) is configured to move in the body (2) in a longitudinal direction between a forward position, at which the cannula (10) is completely extended from the body (2), and a retracted position, at which the cannula (10) is partially inserted in the body (2), and
wherein the elastic means (6, 5) are configured to exert a thrust on the first carriage (3) and on the second carriage (4) in the direction of the respective forward position, and
wherein the loading mechanism (7) comprises a thrust element (13) for both the first carriage (3) and the second carriage (4), which is constrained to the body (2) and is configured to be moved by the operator with respect to the body (2), in a longitudinal direction, between an inactive position and a loading position, and wherein the displacement of the thrust element (13) into the loading position results in the retraction of at least one of the first carriage (3) and the second carriage (4) into the retracted position,
**characterised in that** the thrust element (13) comprises a transverse guide (14') that is orthogonal to the longitudinal axis (X) and the loading mechanism (7) comprises a slider (14) mounted on the thrust element (13) and movable with respect thereto in said transverse guide (14'), in both directions, and the body (2) comprises a longitudinal guide (15) branched into a first branch (15') and a second branch (15"), and wherein the slider (14) is configured to selectively engage the first branch (15') or the second branch (15") by sliding in said transverse guide (14') in response to the retraction of the thrust element (13) into the loading position, thus resulting in the retraction of the second carriage (4) or the first carriage (3), respectively.

2. The gun for biopsies (1) according to claim 1, wherein the slider (14) is configured to engage the first branch (15') of the longitudinal guide (15) in response to a first retraction of the thrust element (13) into the loading position and wherein the slider (14) is configured to engage the second branch (15") of the longitudinal guide (15) in response to a second retraction of the thrust element (13) into the loading position, subsequent in time to the first retraction.

3. The gun for biopsies (1) according to claim 1 or claim 2, wherein the retracted position of the thrust element (13), with the slider (14) engaged in the first branch (15') of the longitudinal guide (15), corresponds to the second carriage (4) in the retracted position.

4. The gun for biopsies (1) according to any one of preceding claims 1-3, wherein the retracted position of the thrust element (13), with the slider (14) engaged in the second branch (15") of the longitudinal guide (15), corresponds to the first carriage (3) in the retracted position.

5. The gun for biopsies (1) according to any one of preceding claims 1-4, wherein at least one of the first carriage (3) and the second carriage (4) is shaped with a wedge-shaped portion (16', 16") which, together with the longitudinal guide (15), defines the path of the slider (14).

6. The gun for biopsies (1) according to any one of preceding claims 1-5, wherein the longitudinal guide (15) comprises a distal end (17) from which the first branch (15') and the second branch (15") branch off, and wherein the distal end (17) is triangular and the vertex (18) is occupied by the slider (14) when the thrust element (13) is in the respective inactive position, and the slider (14) is configured to slidably and selectively engage one of the sides of the vertex of the longitudinal guide (15) when the thrust element (13) is pushed by the user toward the loading position.

7. The gun for biopsies (1) according to claim 6, wherein the angle at the vertex (18) of the distal end (17) of the longitudinal guide (15) is asymmetrical with respect to the longitudinal axis (X).

8. The gun for biopsies (1) according to claim 6 or claim 7, wherein the two sides (18', 18") of the vertex (18) of the distal end (17) of the longitudinal guide (15) have different lengths.

9. The gun for biopsies (1) according to any one of preceding claims 1-8, wherein the thrust element (13) is a single element functionally combined with both the first carriage (3) and the second carriage (4), to cause their retraction, and is configured to be constrained to the body (2) telescopically, in the intermediate position between the body (2) and the needle (9).

10. The gun for biopsies (1) according to any one of preceding claims 1-9, wherein the loading mechanism (7) comprises a selector (19) with a function of stopping the carriages (3, 4), which is interposed between the first carriage (3) and the second carriage (4) on one side and the body (2) on the other side, which is configured to be moved by the user with respect to the body (2) between a first position corresponding to a reduced travel of the first carriage (3) and the second carriage (4), and a second position corresponding to an extended travel of the first carriage (3) and the second carriage (4).

11. The gun for biopsies (1) according to any one of preceding claims 1-10, wherein the loading mechanism (7) comprises an elastic element (20) for counteracting the movement of the thrust element (13), configured to return the thrust element (13) to the respective initial position.

12. The gun for biopsies (1) according to any one of preceding claims 1-11, comprising a first button (A) configured for triggering or firing the gun (1) in automatic mode, the pressure of which by the operator is configured to cause the automatic forward movement of both carriages (3, 4) to the forward position, and a second button (S) configured for triggering or firing the gun (1) in semi-automatic mode, the pressure of which by the operator is configured to cause only the first carriage (3) to move forward to the forward position.

13. The gun for biopsies (1) according to any one of preceding claims 1-12, comprising at least two flexible and elastic fins (21, 22), which extend in a cantilevered way from the inner surface of the body (2) or from a plate (23) housed in the body (2), and wherein each fin (21, 22) configures an undercut with one of the two carriages (3, 4) configured to hold it in the retracted position, by overcoming the thrust of the elastic means (5, 6) combined with the two carriages (3, 4), and wherein the at least one button (A, S) for triggering or firing the gun (1), when pressed by the operator, is configured to engage at least one of the two fins (21, 22) and cause them to bend, thus releasing at least one of the carriages (3, 4) which moves to the forward position.

14. The gun for biopsies (1) according to any one of preceding claims 1-13, wherein the first carriage (3) and the second carriage (4) are arranged in the body (2) side by side and move parallel to the longitudinal axis (X).

## Patentansprüche

1. Pistole (1) für Biopsien, umfassend einen Körper (2), der von einem Bediener gehalten werden kann, einen ersten Schlitten (3) und einen zweiten Schlitten (4), die im Körper (2) untergebracht sind, elastische Mittel (6, 5), die mit dem ersten Schlitten (3) und dem zweiten Schlitten (4) kombiniert sind, einen Lademechanismus (7) der Pistole und mindestens einen Taster (A, S) zum Auslösen oder Abfeuern der Pistole (1) sowie eine Nadel (9), die sich freitragend vom Körper (2) entlang einer Längsachse (X) erstreckt,
wobei die Nadel (9) einen an dem ersten Schlitten (3) befestigten Stilett (11) und eine an dem zweiten Schlitten (4) befestigte Kanüle (10) umfasst,
wobei der Stilett (11) ein spitzes Ende aufweist, an dem sich ein Sammelhohlraum (12) zum Sammeln einer Gewebeprobe befindet, und
wobei das Stilett (11) in der Kanüle (10) aufgenommen ist und in dieser gleiten kann, sodass der Sammelhohlraum (12) aus der Kanüle (10) herauskommen und mit einem Guillotineeffekt in diese zurückgezogen werden kann, und
wobei der erste Schlitten (3) derart konfiguriert ist, dass er sich im Körper (2) in einer Längsrichtung zwischen einer vorderen Stellung, in der das Stilett (11) vollständig aus dem Körper (2) herausgefahren ist und zumindest teilweise aus der Kanüle (10) austritt, und einer zurückgezogenen Stellung, in der das Stilett (11) teilweise in den Körper (2) eingeführt ist und sich der Sammelhohlraum (12) in der Kanüle (10) befindet, bewegt und
wobei der zweite Schlitten (4) derart konfiguriert ist, dass er sich in dem Körper (2) in einer Längsrichtung zwischen einer vorderen Stellung, in der die Kanüle (10) vollständig aus dem Körper (2) herausgefahren ist, und einer zurückgezogenen Stellung, in der die Kanüle (10) teilweise in den Körper (2) eingeführt ist, bewegt, und
wobei die elastischen Mittel (6, 5) dazu ausgelegt sind, eine Schubkraft auf den ersten Schlitten (3) und auf den zweiten Schlitten (4) in Richtung der jeweiligen Vorwärtsstellung auszuüben, und
wobei der Lademechanismus (7) ein Schubelement (13) sowohl für den ersten Schlitten (3) als auch für den zweiten Schlitten (4) umfasst, das an dem Körper (2) befestigt ist und dazu ausgelegt ist, vom Bedienpersonal in Längsrichtung zwischen einer inaktiven Stellung und einer Ladestellung relativ zu dem Körper (2) bewegt zu werden, und
wobei die Verschiebung des Schubelements (13) in die Ladestellung ein Zurückziehen mindestens eines der ersten Schlitten (3) und zweiten Schlitten (4) in die zurückgezogene Stellung bewirkt,
**dadurch gekennzeichnet, dass** das Schubelement (13) eine zur Längsachse (X) orthogonale Querführung (14') umfasst und der Lademechanismus (7) einen auf dem Schubelement (13) montierten und in Bezug zu diesem in der Querführung (14') in beiden Richtungen beweglichen Schieber (14) umfasst und der Körper (2) eine Längsführung (15) umfasst, die in einen ersten Zweig (15') und einen zweiten Zweig (15") verzweigt ist, und wobei der Schieber (14) so konfiguriert ist, dass er durch Gleiten in der Querführung (14') in Reaktion auf das Zurückziehen des Schubelements (13) in die Ladestellung wahlweise in den ersten Zweig (15') oder den zweiten Zweig (15") eingreift, wodurch das Zurückziehen jeweils des zweiten Schlittens (4) oder des ersten Schlittens (3) bewirkt wird.

2. Pistole (1) für Biopsien gemäß Anspruch 1, wobei der Schieber (14) zum Eingreifen in den ersten Zweig (15') der Längsführung (15) in Reaktion auf ein erstes Zurückziehen des Schubelements (13) in die Ladestellung konfiguriert ist, und wobei der Schieber (14) zum Eingreifen in den zweiten Zweig (15") der Längsführung (15) in Reaktion auf ein zweites, zeitlich auf das erste Zurückziehen folgendes Zurückziehen des Schubelements (13) in die Ladestellung konfiguriert ist.

3. Pistole (1) für Biopsien nach Anspruch 1 oder Anspruch 2, wobei die zurückgezogene Stellung des Schubelements (13), in der der Schieber (14) in den ersten Zweig (15') der Längsführung (15) eingreift, dem zweiten Schlitten (4) in der zurückgezogenen Stellung entspricht.

4. Pistole (1) für Biopsien gemäß einem der vorstehenden Ansprüche 1-3,
wobei die zurückgezogene Stellung des Schubelements (13), in der der Schieber (14) in den zweiten Zweig (15") der Längsführung (15) eingreift, dem ersten Schlitten (3) in der zurückgezogenen Stellung entspricht.

5. Pistole (1) für Biopsien gemäß einem der vorstehenden Ansprüche 1-4,
wobei mindestens einer der ersten Schlitten (3) und der zweiten Schlitten (4) mit einem keilförmigen Abschnitt (16', 16") geformt ist, der zusammen mit der Längsführung (15) den Weg des Schiebers (14) definiert.

6. Pistole (1) für Biopsien gemäß einem der vorstehenden Ansprüche 1-5,
wobei die Längsführung (15) ein distales Ende (17) aufweist, von dem der erste Zweig (15') und der zweite Zweig (15") abzweigen, und wobei das distale Ende (17) dreieckig ist und der Scheitelpunkt (18) vom Schieber (14) besetzt ist, wenn sich das Schubelement (13) in der jeweiligen inaktiven Stellung befindet, und der Schieber (14) derart konfiguriert ist, dass er gleitend und selektiv mit einer der Seiten des Scheitelpunkts der Längsführung (15) in Eingriff kommt, wenn das Schubelement (13) vom Benutzer in Richtung der Ladestellung gedrückt wird.

7. Pistole (1) für Biopsien gemäß Anspruch 6, wobei der Winkel am Scheitelpunkt (18) des distalen Endes (17) der Längsführung (15) in Bezug auf die Längsachse (X) asymmetrisch ist.

8. Pistole (1) für Biopsien gemäß Anspruch 6 oder Anspruch 7, wobei die beiden Seiten (18', 18") des Scheitelpunkts (18) des distalen Endes (17) der Längsführung (15) unterschiedliche Längen aufweisen.

9. Pistole (1) für Biopsien gemäß einem der vorstehenden Ansprüche 1-8,
wobei das Schubelement (13) ein Einzelteil ist, das funktional sowohl mit dem ersten Schlitten (3) als auch mit dem zweiten Schlitten (4) verbunden ist, wodurch deren Zurückziehen bewirkt wird, und derart konfiguriert ist, dass es teleskopartig an dem Körper (2) in der Zwischenstellung zwischen dem Körper (2) und der Nadel (9) arretiert werden kann.

10. Pistole (1) für Biopsien gemäß einem der vorstehenden Ansprüche 1-9,
wobei der Lademechanismus (7) einen Selektor (19) mit einer Funktion zum Anhalten der Schlitten (3, 4) umfasst, der zwischen dem ersten Schlitten (3) und dem zweiten Schlitten (4) auf der einen Seite und dem Körper (2) auf der anderen Seite angeordnet ist und der dazu konfiguriert ist, vom Benutzer in Bezug auf den Körper (2) zwischen einer ersten Stellung, die einem reduzierten Verfahrweg des ersten Schlittens (3) und des zweiten Schlittens (4) entspricht, und einer zweiten Stellung, die einem verlängerten Verfahrweg des ersten Schlittens (3) und des zweiten Schlittens (4) entspricht, bewegt zu werden.

11. Pistole (1) für Biopsien gemäß einem der vorstehenden Ansprüche 1-10,
wobei der Lademechanismus (7) ein elastisches Element (20) zur Entgegenwirkung der Bewegung des Schubelements (13) umfasst, das zum Zurückführen des Schubelements (13) in die jeweilige Ausgangsstellung konfiguriert ist.

12. Pistole (1) für Biopsien gemäß einem der vorstehenden Ansprüche 1-11,
umfassend einen ersten Taster (A), der zum Auslösen oder Abfeuern der Pistole (1) im automatischen Modus konfiguriert ist, wobei dessen Druck durch den Bediener dazu konfiguriert ist, die automatische Vorwärtsbewegung beider Schlitten (3, 4) in die Vorwärtsstellung zu bewirken, und einen zweiten Taster (S) zum Auslösen oder Abfeuern der Pistole (1) im halbautomatischen Modus, dessen Druck durch den Bediener dazu konfiguriert ist, nur den ersten Schlitten (3) in die Vorwärtsstellung zu bewegen.

13. Pistole (1) für Biopsien gemäß einem der vorstehenden Ansprüche 1-12,
umfassend mindestens zwei flexible und elastische Stege (21, 22), die sich freitragend von der Innenfläche des Körpers (2) oder von einer im Körper (2) aufgenommenen Platte (23) erstrecken, und wobei jeder Steg (21, 22) eine Hinterschneidung mit einem der beiden Schlitten (3, 4) bildet, die zum Halten derselben in der zurückgezogenen Stellung durch Überwindung des Drucks der elastischen Mittel (5, 6) in Verbindung mit den beiden Schlitten (3, 4) konfiguriert ist, und wobei der mindestens eine Taster (A, S) zum Auslösen oder Abfeuern der Pistole (1 ), sofern er vom Bediener gedrückt wird, derart konfiguriert ist, dass er mit zumindest einem der beiden Stege (21, 22) in Eingriff kommt und deren Biegung bewirkt, wodurch zumindest einer der Schlitten (3, 4) freigegeben wird und sich in die vordere Stellung bewegt.

14. Pistole (1) für Biopsien gemäß einem der vorstehenden Ansprüche 1-13,
wobei der erste Schlitten (3) und der zweite Schlitten (4) nebeneinander im Körper (2) angeordnet sind und sich parallel zur Längsachse (X) bewegen.

## Revendications

1. Un pistolet (1) pour biopsies, comprenant un corps (2) pouvant être tenu par un opérateur, un premier chariot (3) et un second chariot (4) logés dans le corps (2), des moyens élastiques (6, 5) associés au premier chariot (3) et au second chariot (4), un mécanisme de chargement (7) du pistolet, et au moins un bouton (A, S) pour déclencher ou tirer le pistolet (1), et une aiguille (9) s'étendant en porte-à-faux à partir du corps (2) le long d'un axe longitudinal (X),
dans lequel l'aiguille (9) comprend un mandrin (11) contraint au premier chariot (3) et une canule (10) contrainte au second chariot (4),
dans lequel le mandrin (11) a une extrémité pointue en correspondance de laquelle il y a un creux collecteur (12) pour collecter un échantillon de tissu, et
dans lequel le mandrin (11) est logé dans la canule (10), et qui peut y coulisser à l'intérieur, de manière à ce que le creux collecteur (12) puisse sortir de la canule (10) et s'y rétracter avec un effet guillotine, et
dans lequel le premier chariot (3) est configuré pour se déplacer dans le corps (2) dans une direction longitudinale entre une position avant, au niveau de laquelle le mandrin (11) est complètement étendue hors du corps (2) et sort au moins partiellement de la canule (10), et une position arrière, au niveau de laquelle le mandrin (11) est partiellement inséré dans le corps (2) et le creux collecteur (12) est dans la canule (10), et
dans lequel le second chariot (4) est configuré pour se déplacer dans le corps (2) dans une direction longitudinale entre une position avant, au niveau de laquelle la canule (10) est complètement étendue hors du corps (2), et une position arrière, au niveau de laquelle la canule (10) est partiellement insérée dans le corps (2), et
dans lequel les moyens élastiques (6, 5) sont configurés pour exercer une poussée sur le premier chariot (3) et sur le second chariot (4) en direction de la position avant respective, et
dans lequel le mécanisme de chargement (7) comprend un élément de poussée (13) pour à la fois le premier chariot (3) et le second chariot (4), lequel est contraint au corps (2) et est configuré pour être déplacé par l'opérateur par rapport au corps (2) dans une direction longitudinale, entre une position inactive et une position de chargement, et
dans lequel le déplacement de l'élément de poussée (13) dans la position de chargement entraîne la rétraction d'au moins un parmi le premier chariot (3) et le second chariot (4) dans la position arrière,
**caractérisé en ce que** l'élément de poussée (13) comprend un guide transversal (14') qui est orthogonal à l'axe longitudinal (X) et le mécanisme de chargement (7) comprend un curseur (14) monté sur l'élément de poussée (13) et mobile par rapport au même dans ledit guide transversal (14'), dans les deux directions, et le corps (2) comprend un guide longitudinal (15) ramifié en une première branche (15') et en une seconde branche (15"), et dans lequel le curseur (14) est configuré pour engager sélectivement la première branche (15') ou la seconde branche (15") en coulissant dans ledit guide transversal (14') en réponse à la rétraction de l'élément de poussée (13) dans la position de chargement, entraînant ainsi respectivement la rétraction du second chariot (4) ou du premier chariot (3).

2. Le pistolet (1) pour biopsies selon la revendication 1, dans lequel le curseur (14) est configuré pour engager la première branche (15') du guide longitudinal (15) en réponse à une première rétraction de l'élément de poussée (13) dans la position de chargement et dans lequel le curseur (14) est configuré pour engager la seconde branche (15") du guide longitudinal (15) en réponse à une second rétraction de l'élément de poussée (13) dans la position de chargement, consécutivement à la première rétraction.

3. Le pistolet (1) pour biopsies selon la revendication 1 ou la revendication 2, dans lequel la position arrière de l'élément de poussée (13), avec le curseur (14) engagé dans la première branche (15') du guide longitudinal (15), correspond au second chariot (4) dans la position arrière.

4. Le pistolet (1) pour biopsies selon l'une quelconque des revendications précédentes 1-3,
dans lequel la position arrière de l'élément de poussée (13), avec le curseur (14) engagé dans la seconde branche (15") du guide longitudinal (15), correspond au premier chariot (3) dans la position arrière.

5. Le pistolet (1) pour biopsies selon l'une quelconque des revendications précédentes 1-4,
dans lequel au moins un entre le premier chariot (3) et le second chariot (4) est formé avec une portion en forme de coin (16', 16") qui définit, conjointement avec le guide longitudinal (15), le parcours du curseur (14).

6. Le pistolet (1) pour biopsies selon l'une quelconque des revendications précédentes 1-5,
dans lequel le guide longitudinal (15) comprend une extrémité distale (17) à partir de laquelle la première branche (15') et la second branche (15") se ramifient, et dans lequel l'extrémité distale (17) est triangulaire et le sommet (18) est occupé par le curseur (14) lorsque l'élément de poussée (13) est dans la position inactive respective, et le curseur (14) est configuré pour engager de manière coulissante et sélective l'un des côtés du sommet du guide longitudinal (15) lorsque l'élément de poussée (13) est poussé par l'utilisateur vers la position de chargement.

7. Le pistolet (1) pour biopsies selon la revendication 6, dans lequel l'angle au sommet (18) de l'extrémité distale (17) du guide longitudinal (15) est asymétrique par rapport à l'axe longitudinal (X).

8. Le pistolet (1) pour biopsies selon la revendication 6 ou la revendication 7, dans lequel les deux côtés (18', 18") du sommet (18) de l'extrémité distale (17) du guide longitudinal (15) ont des différentes longueurs.

9. Le pistolet (1) pour biopsies selon l'une quelconque des revendications précédentes 1-8,
dans lequel l'élément de poussée (13) est un élément individuel fonctionnellement associé à la fois au premier chariot (3) et au second chariot (4) pour provoquer leur rétraction et est configuré pour être contraint au corps (2) de manière télescopique, dans la position intermédiaire entre le corps (2) et l'aiguille (9).

10. Le pistolet (1) pour biopsies selon l'une quelconque des revendications précédentes 1-9,
dans lequel le mécanisme de chargement (7) comprend un sélecteur (19) avec une fonction d'arrêt des chariots (3, 4) et interposé entre le premier chariot (3) et le second chariot (4) d'un côté et le corps (2) de l'autre, qui est configuré pour être déplacé par l'utilisateur par rapport au corps (2) entre une première position, correspondant à une course réduite du premier chariot (3) et du second chariot (4), et une second position correspondant à une course étendue du premier chariot (3) et du second chariot (4).

11. Le pistolet (1) pour biopsies selon l'une quelconque des revendications précédentes 1-10,
dans lequel le mécanisme de chargement (7) comprend un élément élastique (20) pour contraster le mouvement de l'élément de poussée (13), configuré pour ramener l'élément de poussée (13) dans la position initiale respective.

12. Le pistolet (1) pour biopsies selon l'une quelconque des revendications précédentes 1-11,
comprenant un premier bouton (A) configuré pour déclencher ou tirer le pistolet (1) en mode automatique, dont la pression exercée par l'opérateur est configurée pour provoquer le déplacement automatique vers l'avant des deux chariots (3, 4) vers la position avant, et un second bouton (S) configuré pour déclencher ou tirer le pistolet (1) en mode semi-automatique, dont la pression exercée par l'opérateur est configurée pour provoquer uniquement le déplacement vers l'avant du premier chariot (3) vers la position avant.

13. Le pistolet (1) pour biopsies selon l'une quelconque des revendications précédentes 1-12,
comprenant au moins deux ailettes flexibles et élastiques (21, 22) s'étendant en porte-à-faux à partir de la surface interne du corps (2) ou d'une plaque (23) logée dans le corps (2), et dans lequel chaque ailette (21, 22) configure une contre-dépouille avec l'un des deux chariots (3, 4) configurée pour le maintenir dans la position arrière, en surmontant la poussée des moyens élastiques (5, 6) associés aux deux chariots (3, 4), et dans lequel l'au moins un bouton (A, S) pour déclencher ou tirer le pistolet (1), lorsque pressé par l'opérateur, est configuré pour engager au moins l'une des deux ailettes (21, 22) et pour les faire plier, libérant donc au moins l'un des chariots (3, 4) qui se déplace vers la position avant.

14. Le pistolet (1) pour biopsies selon l'une quelconque des revendications précédentes 1-13,
dans lequel le premier chariot (3) et le second chariot (4) sont disposés dans le corps (2) côte à côte et se déplacent parallèlement à l'axe longitudinal (X).
